# EUROPÄISCHE PATENTANMELDUNG

(11) **EP 3 574 779 A2**
(43) Veröffentlichungstag der Anmeldung: **04.12.2019**
(21) Anmeldenummer: 19176138.6
(22) Anmeldetag: 23.05.2019
(51) Int. Cl.: A24F 47/00, A61M 15/06

(54) **ANORDNUNG FÜR EINEN INHALATOR**

(30) Priorität: 28.05.2018 DE 102018112699; 08.11.2018 DE 102018127927
(71) Anmelder: Hauni Maschinenbau GmbH, 21033 Hamburg (DE)
(72) Erfinder: WOIAS, Peter, 79100 Freiburg (DE); GOLDSCHMIDTBÖING, Frank, 77799 Ortenberg (DE); PELZ, Uwe, 79227 Schallstadt (DE); GHANAM, Muhannad, 79114 Freiburg (DE); JAKLIN, Jan, 79098 Freiburg (DE); RATH, Sonali, 91560 Heilsbronn (DE)
(74) Vertreter: Müller Verweyen

(57) **Zusammenfassung**

Eine Anordnung (1) für einen Inhalator (27) umfasst mindestens einen elektrischen Verdampfer (3, 3a, 3b, 3c, 3d) zum Verdampfen von dem Verdampfer (3, 3a, 3b, 3c, 3d) zugeführter Flüssigkeit (2) und zur Zugabe der verdampften Flüssigkeit (2) zu einem durch den Inhalator (27) strömenden Luftstrom (5) zur Bildung eines Aerosols (6) und eine Durchfluss-Messanordnung (100) zur Messung des Volumen- und/oder Massenstroms des durch den Inhalator (27) strömenden Luftstroms (5). Die Durchfluss-Messanordnung (100) umfasst eine Heizeinrichtung (63), einen stromabwärts der Heizeinrichtung (63) angeordneten nachgeschalteten Temperatursensor (53) zur Messung einer Luftausgangstemperatur und eine elektronische Steuerungsvorrichtung (29), wobei die elektronische Steuerungsvorrichtung (29) zur Ermittlung des Volumen- und/oder Massenstroms des durch den Inhalator (27) strömenden Luftstroms (5) auf der Grundlage einer Temperaturdifferenz zwischen der Luftausgangstemperatur und einer Lufteingangstemperatur des Luftstroms (5) stromaufwärts der Heizeinrichtung (63) eingerichtet ist.

## Beschreibung

Die vorliegende Erfindung betrifft eine Anordnung für einen Inhalator, umfassend mindestens einen elektrischen Verdampfer zum Verdampfen von dem Verdampfer zugeführter Flüssigkeit und zur Zugabe der verdampften Flüssigkeit zu einem durch den Inhalator strömenden Luftstrom zur Bildung eines Aerosols, und eine Durchfluss-Messanordnung zur Messung des Volumen- und/oder Massenstroms des durch den Inhalator strömenden Luftstroms. Die vorliegende Erfindung betrifft außerdem ein Basisteil für einen Inhalator und einen Inhalator.

Im Stand der Technik ist eine Vielzahl von Inhalatoren beziehungsweise elektronischer Zigarettenprodukte mit einer solchen Anordnung bekannt.

Übliche Inhalatoren, beispielsweise elektronische Zigarettenprodukte, werden beispielsweise durch einen Unterdruckschalter aktiviert. Sobald der Nutzer durch ziehen an einem Mundende des Inhalators einen Unterdruck relativ zum Umgebungsdruck erzeugt, wird die Verdampfung der Flüssigkeit aktiviert und diese dem Luftstrom als Aerosol beziehungsweise Dampf zugegeben. Die Flüssigkeit umfasst Aroma- und/oder Wirkstoffe, insbesondere Nikotin, die dem Nutzer mit dem Aerosol beziehungsweise dem Dampf verabreicht werden. Gerät der Unterdruck unter eine Schaltschwelle, wird die Verdampfung beendet. Der Verdampfungsvorgang ist somit unabhängig von der Stärke des erzeugten Unterdrucks, solange die Schaltschwelle über- oder unterschritten ist.

Der Konsument einer konventionellen Zigarette erwartet jedoch ein anderes Verhalten. Bei einer konventionellen Zigarette kann über die Stärke des Zuges die Verbrennung des Tabaks beziehungsweise des Tabakproduktes und somit die Aufnahme von Rauch gesteuert werden. Eine solche Möglichkeit wäre auch für ein elektronisches Zigarettenprodukt beziehungsweise einen Inhalator wünschenswert.

Um der Stärke des Zuges des Konsumenten Rechnung zu tragen, weisen Inhalatoren gemäß dem Stand der Technik Durchfluss-Messanordnungen zur Messung des Volumen- und/oder Massenstroms des durch den Inhalator strömenden Luftstroms auf, siehe beispielsweise DE 10 2009 029 768 B4. Der Nachteil der bekannten Messanordnungen ist die Einschränkung der Messbereiche. Die Unterdruckschalter arbeiten lediglich bei vergleichsweise starken Unterdrücken zuverlässig, während die Durchfluss-Messanordnungen vorrangig bei vergleichsweise kleinen Unterdrücken, Druckdifferenzen beziehungsweise Strömungsgeschwindigkeiten zuverlässig arbeiten. Ferner sind bekannte mechanische Elemente umfassende Durchfluss-Messanordnungen wie Flügelrad-Anemometer, siehe beispielsweise EP 2 563 172 B1, aufwendig, teuer und anfällig für Verschmutzungen und eignen sich nur bedingt zur Erfassung absoluter Messwerte, beispielsweise zum Einschalten des Inhalators.

Die Aufgabe der Erfindung besteht darin, eine Anordnung für einen Inhalator bereitzustellen, der eine verbesserte und zuverlässige Durchflussmessung erlaubt.

Die Erfindung löst diese Aufgabe mit den Merkmalen der unabhängigen Ansprüche.

Erfindungsgemäß wird vorgeschlagen, dass die Durchfluss-Messanordnung eine Heizeinrichtung, einen stromabwärts der Heizeinrichtung angeordneten nachgeschalteten Temperatursensor zur Messung einer Luftausgangstemperatur und eine elektronische Steuerungsvorrichtung umfasst, und die elektronische Steuerungsvorrichtung zur Ermittlung des Volumen- und/oder Massenstroms des durch den Inhalator strömenden Luftstroms auf der Grundlage einer Temperaturdifferenz zwischen der Luftausgangstemperatur und einer Lufteingangstemperatur des Luftstroms stromaufwärts der Heizeinrichtung eingerichtet ist.

Die erfindungsgemäße Anordnung erlaubt eine genaue Durchflussmessung auf thermischer Grundlage. Die Lufteingangstemperatur ist eine stromaufwärts der Heizeinrichtung vorliegende Temperatur und entspricht beispielsweise der Umgebungstemperatur. Die Heizeinrichtung ist dazu eingerichtet, Luft auf die Luftausgangstemperatur zu erwärmen, wobei die erwärmte Luft dem stromabwärts der Heizeinrichtung angeordneten nachgeschalteten Temperatursensor zugeführt wird. Die Heizeinrichtung und/oder der Temperatursensor kann beispielsweise einen ohmschen Widerstand und/oder einen Thermistor, beispielsweise einen Heiß- oder Kaltleiter, umfassen.

Die erfindungsgemäße Anordnung ist frei von mechanischen Teilen, die für die Durchflussmessung bewegt werden müssen und ist somit wenig anfällig für Verschmutzung.

Die durch die Luft von dem Heizelement zum nachgeschalteten Temperatursensor transportierte Wärme gibt Aufschluss über den Massen- und/oder Volumenstrom, welcher mit der Luft transportiert wird. Anhand des Massen- und/oder Volumenstroms kann auf die Strömungsgeschwindigkeit, die Zugstärke beziehungsweise den Saugdruck geschlossen werden.

Vorzugsweise umfasst die Durchfluss-Messanordnung einen stromaufwärts der Heizeinrichtung angeordneten vorgeschalteten Temperatursensor zur Messung der Lufteingangstemperatur des Luftstroms, um einen genauen Referenzwert für die Lufteingangstemperatur ermitteln und vorteilhaft die Temperaturdifferenz bestimmen zu können. Die Anordnung wenigstens zweier Temperatursensoren, wobei insbesondere einer der Temperatursensoren stromaufwärts der Heizeinrichtung und einer der Temperatursensoren stromabwärts der Heizeinrichtung angeordnet ist, erlaubt die beliebige Anordnung der Durchfluss-Messanordnung innerhalb des Inhalators. Auch die Anordnung der Durchfluss-Messanordnung nach dem Verdampfer ist denkbar, da die Temperaturdifferenz exakt und zuverlässig gemessen werden kann.

In anderen Ausführungsformen kann auf den vorgeschalteten Temperatursensor verzichtet werden, wobei die Lufteingangstemperatur geschätzt oder anderweitig, beispielsweise über lokale Wetterinformationen, das Internet und/oder mit dem Inhalator informationstechnisch verbundenen Geräten, bereitgestellt werden kann.

Vorteilhaft ist die Heizeinrichtung stromaufwärts von dem oder den Verdampfern des Inhalators angeordnet, um eine Temperatur mit dem oder den Temperatursensoren an Luft ohne zugegebene Flüssigkeit messen zu können. Dies erlaubt aufgrund der bekannten Wärmekapazität von Luft die vorteilhafte Ermittlung des Volumen- und/oder Massenstroms.

Bevorzugt ist die Heizeinrichtung von einem der Verdampfer gebildet, um einen effektiven Aufbau der Durchfluss-Messanordnung mit nur wenigen Komponenten gewährleisten zu können.

Die Erfindung stell des Weiteren eine Anordnung aufweisend mindestens einen elektrischen Verdampfer zum Verdampfen von dem Verdampfer zugeführter Flüssigkeit und zur Zugabe der verdampften Flüssigkeit zu einem durch den Inhalator strömenden Luftstrom zur Bildung eines Aerosols bereit, wobei die Anordnung erfindungsgemäß eine Nacherwärmungsvorrichtung zur Nacherwärmung des Aerosols umfasst, um eine höhere Dampfqualität zu erzielen.

Die Nacherwärmungsvorrichtung ist vorteilhaft stromabwärts des Verdampfers und/oder in Bezug zu dem Verdampfer angeordnet, d.h. dass das Aerosol nach seiner Erzeugung durch Verdampfung der Flüssigkeit am Verdampfer mit der Nacherwärmungsvorrichtung nacherwärmbar bzw. warmhaltbar ist. Das Aerosol bzw. der Dampf beziehungsweise der Luftstrom mit der zugegebenen Flüssigkeit wird nach der Verdampfung der Flüssigkeit durch den Verdampfer mit der Nacherwärmungsvorrichtung erwärmt. Somit kann eine Kondensation vermieden werden und Aroma- und/oder Wirkstoffe, wie beispielsweise Nikotin, können im Aerosol bzw. Dampf zuverlässig von dem Luftstrom transportiert werden.

Durch die Nacherwärmung kann die Tröpfchengröße beeinflusst und die Wirkung der Aroma- und/oder Wirkstoffe optimiert werden. Die Nacherwärmung des Luftstroms bzw. des Aerosols führt vorzugsweise dazu, dass die Luft für den Konsumenten angenehm warm wird und bereits kondensierte Tropfen des Aerosols nachverdampfen und/oder an der gegebenenfalls weiteren Kondensation gehindert werden. Der dem Konsumenten zugeführte Dampf ist somit feiner und weist eine höhere Dampfqualität auf.

Die Heizeinrichtung kann vorteilhaft von der Nacherwärmungsvorrichtung gebildet sein.

Vorzugsweise ist die elektronische Steuerungsvorrichtung zur Ermittlung des Volumen- und/oder Massenstroms des durch den Inhalator strömenden Luftstroms durch Anwendung der Gleichung Δm / Δt = P/(c·ΔT) eingerichtet, wobei Δ m / Δ t der Massenstrom, P die Heizleistung der Heizeinrichtung, ΔT die Temperaturdifferenz zwischen der Luftausgangstemperatur und der Lufteingangstemperatur und c die bekannte oder geschätzte Wärmekapazität der durch die Heizeinrichtung erwärmten Luft (je nach Anordnung der Heizeinrichtung mit oder ohne Aerosol) sind, um eine einfache und exakte Ermittlung des Massenstroms zu erlauben.

Bevorzugt umfasst die Anordnung ein Wattmeter zur Ermittlung der Heizleistung P der Heizeinrichtung, um die erbrachte Heizleistung über ein Zeitintervall messen zu können und somit die der Luft und/oder dem Aerosol beziehungsweise Dampf zugeführten Wärme ermitteln zu können.

Vorteilhaft ist die elektronische Steuerungsvorrichtung zur Steuerung des mindestens einen Verdampfers in Abhängigkeit der gemessenen Durchflussmenge des Luftstroms durch den Inhalator eingerichtet, um dem Konsumenten eine der Zugstärke beziehungsweise dem Saugdruck angepasste Verdampfung von Flüssigkeit und/oder Zugabe von Aroma- und/oder Wirkstoffen zum Luftstrom zu ermöglichen.

Vorzugsweise ist der Verdampfer auf der Grundlage eines Signals von einem Betätigungselement steuerbar, um dem Konsumenten eine individuell einstellbare Verdampfung von Flüssigkeit und/oder Zugabe von Aroma- und/oder Wirkstoffen zum Luftstrom zu ermöglichen. Das Betätigungselement kann beispielsweise ein Mundpresskraftsensor, einen Fingerdruckkraftmesser, Schalter und/oder Taster umfassen. Insbesondere kann ein elektronisches und/oder kapazitives Betätigungselement am Mundende und/oder an einem sich zwischen dem Mundende und einem distalen Ende des Inhalators erstreckenden Tubus vorgesehen sein.

In einer vorteilhaften Ausführungsform sind mindestens die Heizeinrichtung und der nachgeschaltete Temperatursensor in einem Messkanal angeordnet, um eine von der Verdampfung der Flüssigkeit unabhängige Durchflussmessung zu erlauben. Der Messkanal kann von dem Luftkanal beziehungsweise Hauptluftkanal, in welchem die Flüssigkeit als Aerosol zugegeben wird, wenigstens teilweise separat vorgesehen sein, beispielsweise als Bypass. Der Messkanal kann in einer Ausführungsform durch den Luftkanal bzw. einem Abschnitt des Luftkanals stromaufwärts des Verdampfers gebildet sein.

Vorteilhaft ist der mindestens eine elektrische Verdampfer ein mehrkanaliger, insbesondere temperaturgesteuerter Verdampfer, um eine verbesserte und einfach regel- und/oder steuerbare Verabreichung von Aroma- und/oder Wirkstoffe zu ermöglichen. Ein mehrkanaliger Verdampfer kann beispielsweise von einer Verdampfereinheit mit einer Mehrzahl von Verdampfern und/oder mindestens ein Verdampfer mit einer Mehrzahl separat ansteuerbarer, heizbarer beziehungsweise aktivierbarer Bereiche gebildet sein.

Vorteilhaft ist Verdampfungsmenge der verdampften Flüssigkeit, die Nikotinmenge der verdampften Flüssigkeit, die Temperatur des Verdampfers, der Tastgrad des Verdampfers und/oder die Anzahl der aktivierten Verdampfer und/oder Verdampferbereiche einstellbar, steuerbar und/oder regelbar, um eine hohe Aerosol- bzw. Dampfqualität bereitstellen zu können.

Die Verdampfungsmenge, die Nikotinmenge, die Temperatur, der Tastgrad und/oder die Anzahl der aktivierten Verdampfer und/oder Verdampferbereiche kann durch den Konsumenten und/oder elektronisch einstellbar sein. Beispielsweise kann die Verdampfungsmenge, die Nikotinmenge, die Temperatur, der Tastgrad und/oder die Anzahl der aktivierten Verdampfer und/oder Verdampferbereiche abhängig von der Zusammensetzung der Flüssigkeit und/oder den Konsumgewohnheiten des Konsumenten einstellbar sein. Insbesondere ist die Temperatur und/oder der Tastgrad vorzugsweise über die Dauer eines Verdampfungsintervalls zeitweise steigend, zeitweise fallend und/oder zeitweise gleichbleibend.

Die Verdampfungsmenge, die Nikotinmenge, die Temperatur, der Tastgrad und/oder die Anzahl der aktivierten Verdampfer und/oder Verdampferbereiche sind vorzugsweise unabhängig voneinander variierbar, was beispielsweise eine von der Dampfmenge unabhängige Konzentration von Aroma- und/oder Wirkstoffen ermöglicht.

Vorzugsweise weist die Anordnung einen Kraftsensor zur Messung der Fingeranpresskraft oder der Mundpresskraft durch den Konsumenten auf, um eine einfache Einstellbarkeit des Inhalators durch den Konsumenten zu ermöglichen. Beispielsweise ist an einem Gehäuse des Inhalators ein Kraftsensor zur Messung der Fingeranpresskraft und/oder an dem Mundende des Inhalators ein Mundpresskraftsensor zur Messung der Mundpresskraft vorgesehen. Der Kraftsensor stellt vorzugsweise ein Signal zur Einstellung des Inhalators, insbesondere des Verdampfers, bereit und erlaubt somit die Aktivierung, Steuerung und/oder Regelung der Aroma- und/oder Wirkstoffverabreichung.

Bevorzugt ist die Durchfluss-Messanordnung als Eingabeelement zur Eingabe von Information in die elektronische Steuerungsvorrichtung durch den Konsumenten nutzbar, um eine intuitive Steuerung und/oder Regelung des Inhalators zu ermöglichen.

Vorteilhaft ist die Durchfluss-Messanordnung zur Feststellung von Pusten oder Ziehen in den Inhalator durch den Konsumenten als Eingabe eingerichtet, um insbesondere für das Inhalieren atypische Atmungsmuster, beispielsweise eine atypische Atemfrequenz und/oder ein atypisches Atemvolumen, als Eingabe zu kodieren.

Vorzugsweise ist die Durchfluss-Messanordnung zur Aktivierung des Inhalators nutzbar, um eine unbeabsichtigte Aktivierung des Inhalators zu vermeiden. Beispielsweise kann in dieser Ausführungsform auf einen An-/Aus-Schalter oder ähnliches an der Außenseite des Gehäuses des Inhalators verzichtet werden.

Bevorzugt ist die Durchfluss-Messanordnung zur Dampfmengeneinstellung durch den Konsumenten nutzbar, um beispielsweise die Dampfmenge an die Zugstärke des Konsumenten anpassen und ein Raucherlebnis wie bei einer herkömmlichen Zigarette ermöglichen zu können.

In einer bevorzugten Ausführungsform ist eine Blende und/oder ein Bypasskanal zur Regelung der Strömungsverhältnisse an dem mindestens einen Verdampfer vorgesehen, um eine verbesserte Dampfqualität und/oder Durchflussmessung zu ermöglichen.

In einer bevorzugten Ausführungsform ist der Verdampfer wenigstens teilweise siliziumbasiert, vorzugsweise ein Mikro-Elektro-Mechanisches System (MEMS), um einen effektiven und zuverlässigen Verdampfer bereitstellen zu können.

Die Erfindung wird im Folgenden anhand bevorzugter Ausführungsformen unter Bezugnahme auf die beigefügten Figuren erläutert. Dabei zeigt
- Fig. 1: einen Schnitt durch eine Anordnung mit einer Nacherwärmungsvorrichtung;
- Fig. 2: einen Schnitt durch eine Anordnung mit mehreren Nacherwärmungsvorrichtungen;
- Fig. 3: eine perspektive Ansicht einer Anordnung mit ringförmigen Wärmeleitelementen;
- Fig. 4: eine schematische Darstellung eines Inhalators;
- Fig. 5: eine perspektivische Querschnittsansicht einer Verdampfereinheit mit einem Flüssigkeitsspeicher; und
- Fig. 6: eine schematische Darstellung einer Messanordnung zur Durchflussmessung;
- Fig. 7: eine schematische Darstellung eines Inhalators; und
- Fig. 8: eine schematische Darstellung einer Verdampfereinheit.

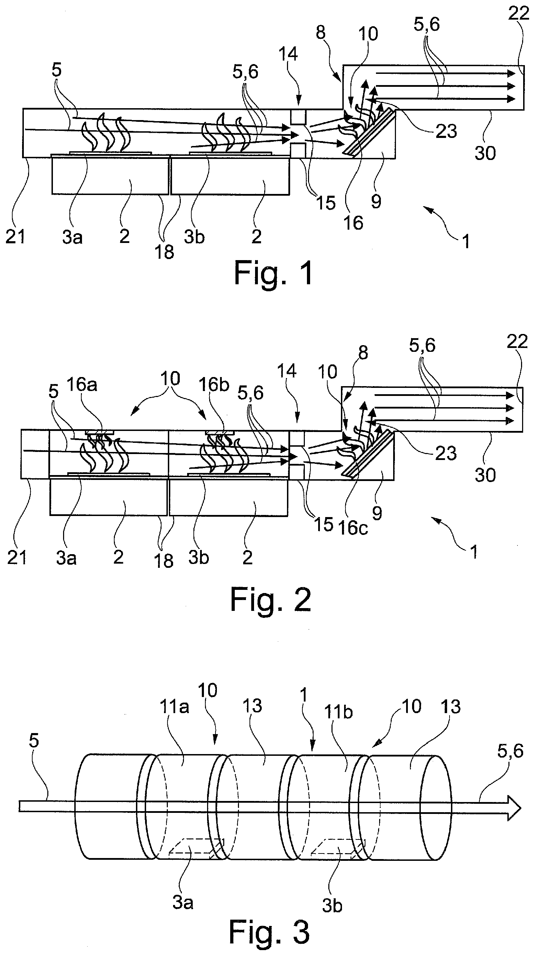

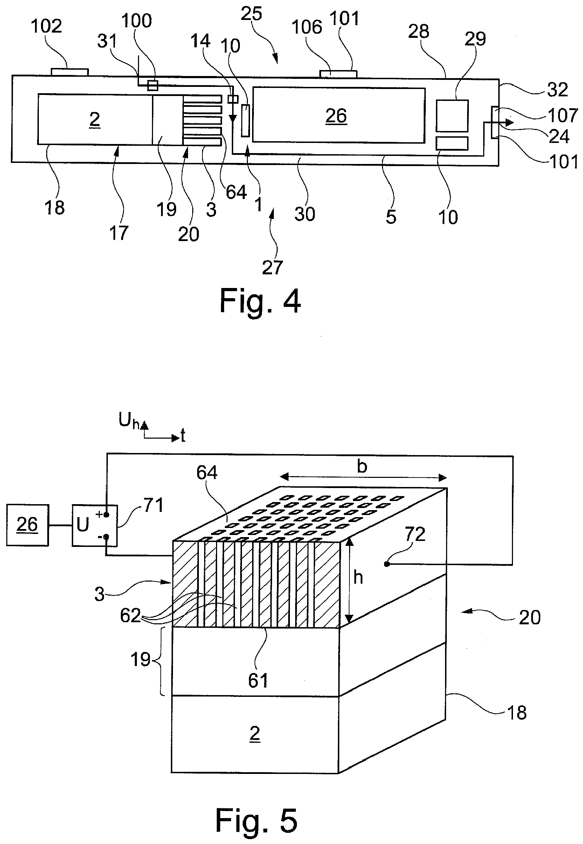

Figur 1 zeigt eine Anordnung 1 mit einer Nacherwärmungsvorrichtung 10. Die Anordnung 1 ist für einen in Figur 4 gezeigten Inhalator 27 vorgesehen. Der Konsument beaufschlagt die Anordnung 1 durch Ziehen an einem Mundende 32 des Inhalators 27 mit einem Unterdruck. Durch den Unterdruck tritt Luft an einem Einlassende 21 der Anordnung 1 in die Anordnung 1 ein und der Unterdruck bewirkt einen Luftstrom 5 durch die Anordnung 1 von dem Einlassende 21 zu einem Auslassende 22, siehe Figur 1.

Die Anordnung 1 umfasst in diesem Beispiel zwei Verdampfer 3a, 3b, um eine Aroma- und/oder Wirkstoffe umfassende Flüssigkeit 2 zu verdampfen. Durch das Verdampfen wird dem Luftstrom 5 Aerosol 6 bzw. Dampf mit den Aroma- und/oder Wirkstoffen zugegeben. Das Aerosol 6 transportiert die von der Flüssigkeit 2 umfassten Aroma- und/oder Wirkstoffe in dem Luftstrom 5 zu dem, dem Mundende 32 zugeordneten Auslassende 22. Zwischen dem Einlassende 21 und dem Auslassende 22 erstreckt sich in der gezeigten Ausführungsform ein Luftkanal 30, durch welchen der Luftstrom 5 geleitet wird. Der Luftstrom 5 befördert das Aerosol 6 vorteilhaft durch den Luftkanal 30. Der Massenstrom Δm / Δt ist die Luftmasse bzw. Luft-/Aerosolmasse Δm, die innerhalb eines Zeitintervalls Δt durch den Luftkanal 30 strömt. Der (Luft-)Massenstrom beeinflusst das Raucherlebnis beziehungsweise die Verabreichung von Aroma- und/oder Wirkstoffen, welche im Aerosol 6 enthalten sind.

Die Verdampfer 3a, 3b sind beispielsweise mit einer in dem Inhalator 27 vorgesehenen elektronischen Steuerungsvorrichtung 29 und/oder einem in dem Inhalator 27 vorgesehen Energiespeicher 26 zur Versorgung mit Energie verbunden. Die Verdampfer 3a, 3b beziehen zur Verdampfung und/oder Wärmeerzeugung vorteilhaft über einen definierten beziehungsweise definierbaren Zeitraum eine Leistung P. Die Verdampfer 3a, 3b bewirken, dass die Temperatur des Luftstroms 5 vorteilhaft stromabwärts eines jeden Verdampfers 3a, 3b höher ist als stromaufwärts des jeweiligen Verdampfers 3a, 3b, d.h. die Verdampfer 3a, 3b bewirken eine Temperaturdifferenz ΔT im Luftstrom 5.

Die Verdampfer 3a, 3b sind entlang des Luftstroms 5 beziehungsweise in dem Luftkanal 30 in Strömungsrichtung hintereinander und voneinander beabstandet angeordnet, d.h. die Verdampfer 3a, 3b sind in Richtung des Luftstroms 5 sequenziell geschaltet. Die Verdampfer 3a, 3b können dazu eingerichtet sein, verschiedene Flüssigkeiten 2 und/oder Flüssigkeit 2 bei verschiedenen Temperaturen zu verdampfen.

Die Flüssigkeit 2 ist in einem oder mehreren, hier beispielhaft zwei, Flüssigkeitsspeichern 18 gespeichert und kann durch eine zwischen dem jeweiligen Verdampfer 3a, 3b und dem entsprechenden Flüssigkeitsspeicher 18 über eine in Figur 5 gezeigte Dochtstruktur 19 dem jeweiligen Verdampfer 3a, 3b zugeführt werden. In einer nicht gezeigten Ausführungsform kann die Anordnung 1 einen Verdampfer 3a, 3b und/oder einen Flüssigkeitsspeicher 18 umfassen.

In der in Figur 1 gezeigten Ausführungsform ist den Verdampfern 3a, 3b eine Drossel 14 nachgeordnet, das heißt die Drossel 14 ist stromabwärts von den Verdampfern 3a, 3b angeordnet. Die Drossel 14 ist eine Verengung des Querschnitts des den Luftstrom 5 zur Verfügung stehenden Volumens. Die Drossel 14 bewirkt somit vorteilhaft eine Einstellung des Zugwiderstandes, des Massenstroms Δm / Δt des Luftstroms 5 durch den Luftkanal 30 und/oder eine Verwirbelung des Luftstroms 5. Die Verwirbelung des Luftstroms 5 kann gezielt genutzt werden, um den Wärmetransport innerhalb des Luftstroms 5 zu beeinflussen und/oder den Luftstrom 5 zu durchmischen, eine turbulente Strömung hervorzurufen bzw. eine laminare Strömung zu unterdrücken. Der so durch die Drossel 14 verbesserte Wärmetransport innerhalb des Luftstroms kann vorzugsweise die Rekondensation des Aerosols 6 und/oder Dampfes vermindern.

Die Drossel 14 ist vorteilhaft dazu eingerichtet, den Querschnitt des Luftkanals 30 zu verringern. Bevorzugt ist die durch die Drossel 14 bewirkte Veränderung des Querschnitts einstellbar. Beispielsweise kann an der Außenseite des Inhalators 27 eine den Konsumenten zugängige Einstellvorrichtung zur Einstellung des Querschnitts der Drossel 14 vorgesehen sein. Die Einstellvorrichtung kann beispielsweise ein Stellrad, Schalter, Taster und/oder Ähnliches umfassen.

Die Drossel 14 und/oder der Zugwiderstand kann in einer Ausführungsform durch den Konsumenten mittels Blasen beziehungsweise Pusten und/oder Saugen beziehungsweise Ziehen einstellbar sein. Eine bestimmte Abfolge von Pusten und/oder Ziehen kann einen entsprechenden Zugwiderstand und/oder eine Einstellung der Drossel 14 kodieren. Die Einstellung der Drossel 14 kann an einer an der Außenseite des Inhalators 27 vorgesehenen Anzeigevorrichtung 102 darstellbar sein.

Bevorzugt ist der Querschnitt der Drossel 14 elektrisch beziehungsweise elektronisch einstellbar. Die Drossel 14 kann beispielsweise ein oder mehrere piezoelektrisches oder induktiv gesteuerte Elemente 15 aufweisen, welche dazu eingerichtet sind, aufgrund elektrischer Signale den Querschnitt der Drossel 14 einzustellen. Die oder das piezoelektrische oder induktive Element 15 ist beziehungsweise sind so angeordnet, dass der Querschnitt des Luftkanals 30 beziehungsweise das dem Luftstrom 5 zur Verfügung stehende Volumen vorteilhaft aufgrund der elektrischen Signale oder deren Ausbleiben veränderlich ist. Beispielsweise kann ein piezoelektrisches oder induktives Element 15 den Luftstrom 5 quer zu der Strömungsrichtung beziehungsweise in Umfangsrichtung umgeben. In einer anderen Ausführungsform können mehrere piezoelektrische und/oder induktive Elemente 15 in Strömungsrichtung auf gleicher Höhe angeordnet sein, um eine Verengung des Querschnitts zu erreichen.

Die Drossel 14 ist beispielsweise mit der in dem Inhalator 27 vorgesehenen elektronischen Steuerungsvorrichtung 29 zur Einstellung des Querschnitts der Drossel 14 verbunden.

In einer weiteren Ausführungsform weist der Inhalator eine Mehrzahl von Luftkanälen 30 auf. Jeder der Luftkanäle 30 kann eine zugeordnete Drossel 14 aufweisen, wobei vorteilhaft jede Drossel 14 vollständig schließbar ist, um den Luftstrom 5 durch den jeweiligen Luftkanal 30 zu unterbinden. Jeder der Luftkanäle ist vorzugsweise einzeln aktivierbar beziehungsweise schaltbar um den Luftstrom 5 durch den jeweiligen Luftkanal 30 zu erlauben oder zu unterbinden. Das Unterbinden des Luftstroms 5 durch einen oder mehreren der Luftkanäle 30 und das Führen des Luftstroms 5 durch einen oder mehrere der Luftkanäle 30 bewirkt die zuverlässige Regulierung des Zugwiderstandes, des Massenstroms und der Aroma- und/oder Wirkstoffzugabe zum Gesamtluftstrom, der sich beispielsweise aus der Summe aller Luftströme 5 durch passierbare beziehungsweise aktivierte Kanäle zusammensetzt.

In der in Figur 1 gezeigten Ausführungsform ist stromabwärts der Drossel 14 eine Nacherwärmungsvorrichtung 10 angeordnet. Die Drossel 14 ist in diesem Beispiel in Strömungsrichtung zwischen den Verdampfern 3a, 3b und der Nacherwärmungsvorrichtung 10 angeordnet. Ein von der Drossel 14 vorteilhaft verwirbelter Luftstrom 5 trifft auf die Nacherwärmungsvorrichtung 10 und ermöglicht eine effektive Erwärmung des Luftstroms 5.

Die Nacherwärmungsvorrichtung 10 umfasst vorteilhaft ein Heizelement 16, welches dazu eingerichtet ist, den auf die Nacherwärmungsvorrichtung 10 treffenden Luftstrom 5 mit darin befindlichen Aerosol 6 bzw. Dampf zu erhitzen. Die Nacherwärmungsvorrichtung 10 ist vorteilhaft von dem Verdampfer 3 verschieden. Der Nacherwärmungsvorrichtung 10 ist vorteilhaft Flüssigkeit 2 nur über den Luftstrom 5 als Aerosol 6 und/oder Dampf zuführbar.

Das Heizelement 16 ist beispielsweise mit der in dem Inhalator 27 vorgesehenen elektronischen Steuerungsvorrichtung 29 und/oder dem in dem Inhalator 27 vorgesehen Energiespeicher 26 zur Versorgung mit Energie verbunden. Das Heizelement 16 bezieht zur Wärmeerzeugung vorteilhaft über einen definierten beziehungsweise definierbaren Zeitraum eine Leistung P. Das Heizelement 16 bewirkt, dass die Temperatur des Luftstroms 5 stromabwärts des Heizelements 16 höher ist als stromaufwärts des Heizelements, d.h. das Heizelement 16 bewirkt eine Temperaturdifferenz ΔT im Luftstrom 5. Die Erhöhung der Temperatur des Luftstroms 5 durch das Heizelement 16 beziehungsweise durch die Nacherwärmungsvorrichtung 10 wirkt vorteilhaft einer Rekondensation des im Luftstrom 5 transportierten Aerosols 6 oder einem Niederschlag dessen am Luftkanal 30 und/oder anderer Komponenten des Inhalators entgegen.

Die Anordnung 1 in Figur 1 umfasst vorteilhaft Luftleitelemente 23, welche dazu eingerichtet sind, den Luftstrom 5 zu leiten und/oder zu verwirbeln. In dieser Ausführungsform umfassen die Luftleitelemente 23 eine Wegbiegung 8 des Luftstroms 5 bzw. des Luftkanals 30 und eine Luftleiteinrichtung 9, beispielsweise ausgebildet als eine Neigung beziehungsweise Anschrägung der Nacherwärmungsvorrichtung 10 bzw. des Heizelements 16 zur Strömungsrichtung. Die der Nacherwärmungsvorrichtung 10 zugeordnete Luftleiteinrichtung 9 bewirkt, dass der Luftstrom 5 unter einem Winkel zwischen 10° und 80°, vorteilhaft zwischen 20° und 70°, vorzugsweise zwischen 30° und 60°, besonders bevorzugt zwischen 40° und 50°, beispielsweise von 45° auf die Nacherwärmungsvorrichtung 10 trifft und die Nacherwärmungsvorrichtung 10 somit den Luftstrom 5 effektiv erwärmen kann.

Vorteilhaft ist die Heiztemperatur des oder der Verdampfer 3 und/oder der Nacherwärmungsvorrichtung 10 unter Berücksichtigung unterschiedlicher Dampfphasen der Bestandteile der Flüssigkeit 2 beziehungsweise des Aerosols 6 einstellbar, steuerbar und/oder regelbar. Bei einer gepulsten Ansteuerung beziehungsweise Beheizung des Verdampfers 3 und/oder der Nacherwärmungsvorrichtung 10 ist eine Anpassung des Tastgrades zur definierten Leistungs- beziehungsweise Wärmeabgabe vorgesehen. Vorteilhaft ist die von dem Verdampfer 3 und/oder der Nacherwärmungsvorrichtung 10 hervorgerufene Temperaturänderung ΔT und/oder die Leistung P einstellbar, um eine hohe Qualität der Zusammensetzung des Aerosols 6 zu ermöglichen.

Figur 2 zeigt eine Anordnung 1 mit mehreren in Strömungsrichtung hintereinander angeordneten Nacherwärmungsvorrichtungen 10. Die gezeigte Ausführungsform wird anhand der Unterschiede zu der Ausführungsform aus Figur 1 erläutert. Die Nacherwärmungsvorrichtungen 10 umfassen jeweils mindestens ein Heizelement 16a, 16b, 16c. Die Heizelemente 16a, 16b, 16c sind in Strömungsrichtung hintereinander und voneinander beabstandet angeordnet. Jedes der Heizelemente 16a, 16b, 16c erwärmt den Luftstrom mit einer Leistung Pi, wobei die Leistung Pi für jedes der Heizelemente 16a, 16b, 16c individuell sein kann, insbesondere individuell für jedes Heizelement 16a, 16b, 16c einstellbar, in Strömungsrichtung steigend und/oder fallend.

Dem in Strömungsrichtung ersten Verdampfer 3a ist eines der Heizelemente 16a gegenüberliegend angeordnet, d.h. das Heizelement 16a ist wenigstens teilweise dem Verdampfer 3a zugewandt und ist quer zur Strömungsrichtung auf der gegenüberliegenden Seite im Luftkanal 30 angeordnet. Stromabwärts von dem ersten Verdampfer 3a ist ein zweiter Verdampfer 3b angeordnet, welchem gegenüber ein weiteres der Heizelemente 16b angeordnet ist. Stromabwärts der Verdampfer 3a, 3b und der Drossel 14 ist ein weiteres der Heizelemente 16c angeordnet. Die Nacherwärmungsvorrichtungen 10 und die Heizelemente 16a, 16b, 16c sind in Strömungsrichtung sequenziell angeordnet und jeder der Nacherwärmungsvorrichtungen 10 ist dazu eingerichtet, die Temperatur des Luftstroms 5 um eine Temperaturdifferenz ΔT zu erhöhen, wobei die Temperaturdifferenz ΔT für jede der Nacherwärmungsvorrichtungen 10 individuell sein kann, insbesondere für jede der Nacherwärmungsvorrichtungen 10 individuell einstellbar, stromabwärts steigend und/oder stromabwärts fallend.

Die verdampfte Flüssigkeit 2 und die im Luftstrom 5 enthaltene Luft dehnt sich bei Verdampfung durch den Verdampfer 3a, 3b entsprechend der durch den Verdampfer 3a, 3b zugeführten Wärme und/oder durch die Verdampfung beziehungsweise die Bildung des Aerosols aus. Somit kommt es zu einer Ausbreitung des Aerosols 6 im Luftkanal 30, insbesondere senkrecht zur Strömungsrichtung zwischen Einlassende 21 und Auslassende 22. Die jeweils einem der Verdampfer 3a, 3b gegenüberliegende Nacherwärmungsvorrichtung 10 beziehungsweise das jeweils einem der Verdampfer 3a, 3b gegenüberliegende Heizelement 16a, 16b ist so angeordnet, dass die Wand des Luftkanal 30 gegenüber der Verdampfer 3a, 3b erwärmt wird, um einen Niederschlag beziehungsweise eine Kondensation des Aerosols 6 zu unterbinden.

Die Nacherwärmungsvorrichtungen 10 beziehungsweise die Heizelemente 16a, 16b, 16c sind in Strömungsrichtung beabstandet und/oder sequenziell angeordnet. Zwischen den Nacherwärmungsvorrichtungen 10 und/oder den Verdampfern 3a, 3b ist der Luftstrom 5 nicht beheizt und kühlt ab. Die Nacherwärmungsvorrichtungen 10 beziehungsweise die Heizelemente 16a, 16b, 16c sind zur Erwärmung des Luftstroms 5 mit jeweils einer Leistung Pi über einen Zeitraum eingerichtet. Die Leistung Pi der Nacherwärmungsvorrichtungen 10 beziehungsweise der Heizelemente 16a, 16b, 16c ist einstellbar und/oder kann gleich oder verschieden, insbesondere in Strömungsrichtung steigend oder abnehmend, sein.

Figur 3 zeigt eine Anordnung 1 mit den Luftstrom 5 umgebenden Wärmeleitelementen 11a, 11b. Der Luftstrom 5 erstreckt sich durch die vorzugsweise ringförmig ausgebildeten Wärmeleitelemete 11a, 11b. In diesem Ausführungsbeispiel sind zwei Wärmeleitelemente 11a, 11b in Strömungsrichtung voneinander beabstandet angeordnet. In anderen Ausführungsformen können ein oder mehr als zwei ringförmige Wärmeleitelemente 11a, 11b vorgesehen sein.

Die Wärmeleitelemente 11a, 11b sind vorteilhaft durch einen der Verdampfer 3a, 3b beheizbar. Jedes Wärmeleitelement 11a, 11b ist vorteilhaft von wenigstens einem der Verdampfer 3a, 3b beheizbar. Beispielsweise ist das in Strömungsrichtung erste Wärmeleitelement 11a von dem in Strömungsrichtung ersten Verdampfer 3a beheizbar und/oder das stromabwärts von dem ersten Wärmeleitelement 11a angeordnete zweite Wärmeleitelement 11b ist von dem stromabwärts vom ersten Verdampfer 3a angeordneten zweiten Verdampfer 3b beheizbar. Dabei wird die von einem der Verdampfer 3a, 3b an das jeweilige Wärmeleitelement 11a, 11b abgegebene Wärme genutzt, um in einem beispielsweise zylinderförmigen Teilabschnitt des Luftkanals 30, beispielsweise eines Hauptluftkanals des Inhalators 27, Kondensation des Aerosols 6 zu vermeiden und den Luftstrom 5 zu erwärmen.

In dieser Ausführungsform sind die Verdampfer 3a, 3b in das jeweilige Wärmeleitelement 11a, 11b eingebettet, d.h. die Wärmeleitelemente 11a, 11b weisen je vorzugsweise eine Aufnahme auf, auf der beziehungsweise in der der jeweilige Verdampfer 3a, 3b aufnehmbar ist. Die Aufnahme kann beispielsweise eine Aussparung sein.

Zwischen den Wärmeleitelementen 11a, 11b können Kühlelemente 13 beziehungsweise Kühlstrecken vorgesehen sein. Die Kühlelemente 13 können zur passiven Kühlung des Aerosols 6 eingerichtet sein oder aktiv, beispielsweise elektrisch kühlbar sein.

Figur 4 zeigt schematisch einen Inhalator 27. Der Inhalator 27, hier ein elektronisches Zigarettenprodukt, umfasst ein Gehäuse 28, in dem ein Luftkanal 30 zwischen mindestens einer Lufteinlassöffnung 31 und einer Luftauslassöffnung 24 an einem Mundende 32 des Zigarettenprodukts 27 vorgesehen ist. Das Mundende 32 des Zigarettenprodukts 27 bezeichnet dabei das Ende, an dem der Konsument zwecks Inhalation zieht und dadurch das Zigarettenprodukt 27 mit einem Unterdruck beaufschlagt und einen Luftstrom 5 in dem Luftkanal 30 erzeugt.

Das Zigarettenprodukt 27 besteht vorteilhaft aus einem Basisteil 25 und einer Verbrauchseinheit 17, die die Verdampfereinheit 20 mit dem Verdampfer 3 und den Flüssigkeitsspeicher 18 umfasst und insbesondere in Form einer auswechselbaren Kartusche ausgebildet ist.

Vorzugsweise ist eine Nacherwärmungsvorrichtung 10 in dem Inhalator 27 vorgesehen. Die Nacherwärmungsvorrichtung 10 ist wenigstens teilweise in der Verbrauchseinheit 17 angeordnet. Die Nacherwärmungsvorrichtung 10 kann auch wenigstens teilweise in dem Basisteil 25 angeordnet sein. Die durch die Einlassöffnung 31 angesaugte Luft wird in dem Luftkanal 30 zu oder entlang mindestens einer Verdampfereinheit 20 geleitet, wobei die Verdampfereinheit wenigstens einen Verdampfer 3, 3a, 3b, 3c, 3d umfasst. Das in Figur 4 gezeigte Beispiel umfasst vorteilhaft eine Anordnung 1 gemäß einer der in Figuren 1 bis 3 gezeigten Ausführungsbeispiele.

Die Verdampfereinheit 20 in Figur 4 ist mit mindestens einem Flüssigkeitsspeicher 18 verbunden oder verbindbar, in dem mindestens eine Flüssigkeit 2 gespeichert ist. Die Verdampfereinheit 20 verdampft Flüssigkeit 2, die ihr aus dem Flüssigkeitsspeicher 18 zugeführt wird, und gibt die verdampfte Flüssigkeit als Aerosol/Dampf an einer Auslassseite 64 in den Luftstrom 5 zu. Ein vorteilhaftes Volumen des Flüssigkeitsspeichers 18 liegt im Bereich zwischen 0,1 ml und 5 ml, vorzugsweise zwischen 0,5 ml und 3 ml, weiter vorzugsweise zwischen 0,7 ml und 2 ml oder 1,5 ml. Die in dem Flüssigkeitsspeicher 18 gespeicherte, zu dosierende Flüssigkeit 2 ist beispielsweise eine Mischung aus 1,2-Propylenglykol, Glycerin, Wasser, mindestens einem Aroma (Flavour) und/oder mindestens einem Wirkstoff insbesondere Nikotin und/oder einem medizinischen Wirkstoff.

Die elektronische Zigarette 27 umfasst des Weiteren einen elektrischen Energiespeicher 26 und eine elektronische Steuerungsvorrichtung 29. Der Energiespeicher 26 ist in der Regel in dem Basisteil 25 angeordnet und kann insbesondere eine elektrochemische Einweg-Batterie oder ein wiederaufladbarer elektrochemischer Akku, beispielsweise ein Lithium-Ionen-Akku, sein. Die elektronische Steuerungsvorrichtung 29 umfasst mindestens eine digitale Datenverarbeitungseinrichtung, insbesondere Mikroprozessor und/oder Mikrocontroller, in dem Basisteil 25 (wie in Figur 4 gezeigt) und/oder in der Verbrauchseinheit 17.

Die Verbrauchseinheit bzw. Kartusche 17 umfasst vorteilhaft einen nichtflüchtigen Datenspeicher zum Speichern von die Verbrauchseinheit bzw. Kartusche 17 betreffender Information bzw. Parameter. Der Datenspeicher kann Teil der elektronischen Steuerungsvorrichtung 29 sein. In dem Datenspeicher ist vorteilhaft Information zur Zusammensetzung der in dem Flüssigkeitsspeicher 18 gespeicherten Flüssigkeit, Information zum Prozessprofil, insbesondere Leistungs-/Temperatursteuerung; Daten zur Zustandsüberwachung bzw. Systemprüfung, beispielsweise Dichtigkeitsprüfung; Daten betreffend Kopierschutz und Fälschungssicherheit, eine ID zur eindeutigen Kennzeichnung der Verbrauchseinheit bzw. Kartusche 17, Seriennummer, Herstelldatum und/oder Ablaufdatum, und/oder Zugzahl (Anzahl der Inhalationszüge durch den Konsumenten) bzw. der Nutzungszeit gespeichert. Der Datenspeicher ist vorteilhaft über Kontakte und/oder Leitungen mit der Steuerungsvorrichtung 29 verbunden oder verbindbar. Der Inhalator 27 umfasst vorteilhaft einen Sensor, beispielsweise einen Druck- oder Strömungssensor beziehungsweise eine Durchfluss-Messanordnung 100 oder einen Druck- oder Strömungsschalter, wobei insbesondere die Steuerungsvorrichtung 29 auf der Grundlage eines von dem Sensor oder Schalter ausgegebenen Sensorsignals feststellen kann, dass ein Konsument am Mundende 32 des Zigarettenprodukts 27 zieht, um zu inhalieren. In diesem Fall steuert die Steuerungsvorrichtung 29 die Verdampfereinheit 20 an, um Flüssigkeit 2 aus dem Flüssigkeitsspeicher 18 als Aerosol/Dampf 6 in den Luftstrom 5 zuzugeben.

An dem Gehäuse 28 ist vorteilhaft wenigstens ein Betätigungselement 101 vorgesehen, siehe Figur 4. Das Betätigungselement 101 kann beispielsweise ein oder mehrere Schalter, Taster, Fingerdruckkraftmesser sein. Insbesondere elektronische und/oder kapazitive Betätigungselemente 101 sind denkbar. Das Betätigungselement 101 kann beispielsweise zur Dosierung der Aroma- und/oder Wirkstoffe, insbesondere Nikotin, und/oder zur Einstellung des Zugwiderstandes eingerichtet sein.

Das Betätigungselement 101 ist vorteilhaft so eingerichtet und angeordnet, um von Konsumenten durch einen Fingerdruck und/oder durch eine Mundpresskraft betätigbar zu sein. Das Betätigungselement 101 ist vorteilhaft als fingerdruckkraftsensitiver Sensor 106 und/oder mundpresskraftsensitiver Sensor 107 ausgebildet. Als Fingerdrucksensor beziehungsweise Kraftsensor 106 ist das Betätigungselement 101 vorteilhaft an einem sich zwischen dem Mundende 32 und einem distalen Ende des Gehäuses 28 erstreckenden Tubus angeordnet und als mundpresskraftsensitiver Sensor beziehungsweise Mundpresskraftsensor 107 ist das Betätigungselement 101 vorteilhaft am Mundende 32 des Gehäuses 28 zur Wahrnehmung der Berührung der Lippen des Konsumenten angeordnet.

Die Messung der Zugstärke beziehungsweise des Saugdrucks kann vorteilhaft das Schalten von in der Ausführungsform nicht gezeigten Bypässen 109 beziehungsweise weiteren Luftkanälen und/oder das Verstellen von Drosseln 14 oder Blenden in einem oder mehreren Luftkanälen 30 ermöglichen.

Der Konsument beziehungsweise Nutzer kann vorteilhaft die Dampfentwicklung beziehungsweise den Luftstrom 5 mit dem Aerosol 6, insbesondere den Massenstrom und/oder die Temperatur, auf eine intuitive Weise steuern. Die Verdampfungsmenge ist vorteilhaft durch das Betätigungselement 101 mit einem Kraftsensor 106 im Griffbereich des Inhalators 27 zur Messung der Anpresskraft der Finger, einstellbar. Vorteilhaft ist die Verdampferleistung P des Verdampfers 3, dessen Dampfrate variabel ist, anhand einer zeitaufgelösten Messung der Betätigung beziehungsweise Griffstärke des Betätigungselements 101 an die Griffstärke anpassbar. Vorzugsweise ist der Tastgrad und/oder die Temperatur des Verdampfers 3 einstellbar, um den Aroma- und/oder Wirkstoffanteil, insbesondere den Nikotinanteil, im Aerosol 6 beziehungsweise in der Dampfphase anhand der zeitaufgelösten Messung des Betätigungselements 101 an die Griffstärke anzupassen.

Die Verdampfungsmenge und/oder der Aroma- und/oder Wirkstoffgehalt, beispielsweise der Nikotingehalt, sind vorteilhaft durch mehrere unabhängige, vorzugsweise intuitive, Methoden, insbesondere die Ermittlung des Saugdrucks mit der Durchfluss-Messanordnung 100 und der Betätigung das Betätigungselement 101, durch den Nutzer steuerbar. Die Durchfluss-Messanordnung 100 beziehungsweise der Druck- oder Strömungssensor kann als Eingabeelement für die elektronische Steuerungsvorrichtung 29 vorgesehen sein.

Vorzugsweise ist die elektronische Steuerungsvorrichtung 29 dazu eingerichtet, den Tastgrad und/oder die Temperatur des Verdampfers 3 vorteilhaft genügend schnell einstellen zu können, um den Aroma- und/oder Wirkstoffanteil, insbesondere den Nikotinanteil, im Aerosol 6 beziehungsweise in der Dampfphase und die Dampfrate beziehungsweise den Massenstrom vorteilhaft genügend schnell, d.h. während eines Zuges, und unabhängig voneinander einstellen zu können und/oder vorteilhaft genügend schnell auf eine zeitaufgelöste Messung der Messanordnung 100 und/oder des Bedienelements 101 reagieren zu können.

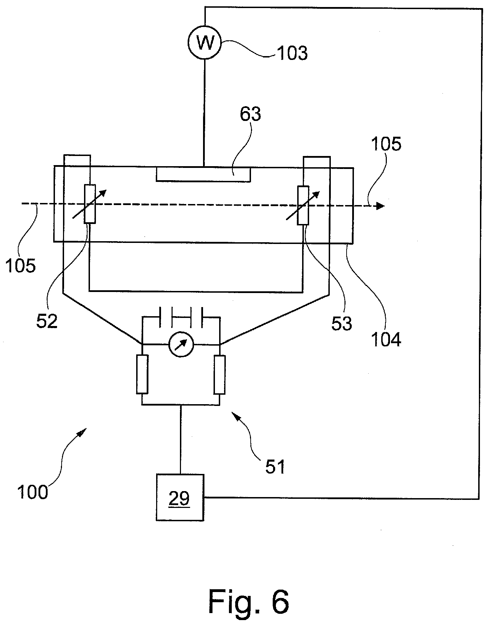

Entlang des Luftkanals 30 sind vorteilhaft eine Mehrzahl von Nacherwärmungsvorrichtungen 10 beispielsweise gemäß einer der Figuren 1 bis 3 und/oder die Messanordnung 100 beispielsweise gemäß Figur 6 angeordnet. Die Nacherwärmungsvorrichtungen 10 sind vorteilhaft mit der Steuerungsvorrichtung 29 verbunden.

In dem Luftkanal 30 kann die Drossel 14, Luftleitelemente 23 und/ oder Blenden einstellbar sein, um Strömungsverhältnisse am Verdampfer 3 zu ändern und/oder einen Messkanal und/oder Bypässe beziehungsweise Nebenluftkanäle zu- oder abzuschalten.

Es können in anderen Ausführungsformen eine Mehrzahl von Verdampfereinheiten 20 und/oder Verdampfern 3, 3a, 3b, 3c, 3d in dem Luftkanal 30 oder einer Mehrzahl von Luftkanälen vorgesehen sein. Vorteilhaft wird dabei eine Mehrzahl von Luftströmen 5 durch eine Mehrzahl von Luftkanälen 30 geführt, wobei jeder der Luftkanäle 30 durch eine Drossel 14 oder Blende aktivierbar beziehungsweise zu öffnen und deaktivierbar beziehungsweise schließbar ist. D.h. durch einen aktiven beziehungsweise offenen Luftkanal 30 kann ein Luftstrom 5 geführt werden und ein inaktiver beziehungsweise geschlossener Luftkanal 30 ist für einen Luftstrom 5 nicht passierbar. Jede Verdampfereinheit 20 und/oder jeder Verdampfer 3, 3a, 3b, 3c, 3d ist so angeordnet, dass Aerosol 6 zu wenigstens einem der Luftkanäle 30 zugebbar ist. Zur Inhalation durch den Konsumenten wird die Summe aller durch die Luftkanäle 30 strömenden Luftströme 5 zum Mundende 32 geleitet.

In einer weiteren Ausführungsform ist es durch eine kanalweise Ansteuerung der Verdampfer 3, 3a, 3b, 3c, 3d und/oder Verdampfereinheit 20 möglich, dass die Verdampfer 3, 3a, 3b, 3c, 3d schnell, beispielsweise in weniger als 1 s, vorzugsweise weniger als 0,2 s, die Verdampfung beginnen und/oder beenden können, um auf das Ziehen des Konsumenten und/oder eine andere Ansteuerung geeignet reagieren zu können beziehungsweise eine direkte Reaktion auf den Nutzerwunsch zu ermöglichen. Durch die vorzugsweise kanalweise Ansteuerung der Verdampfer oder des Verdampfers 3, 3a, 3b, 3c, 3d kann die Verdampfungsrate beziehungsweise der Massenstrom mit einer vorteilhaft geringen Ansprechverzögerung für die Änderung der Verdampfungseigenschaften eingestellt werden. Die Ansprechverzögerung ist abhängig von der Auslegung der Verdampfer 3, 3a, 3b, 3c, 3d, insbesondere der Materialwahl, Geometrie und der thermischen Ankopplung an die Umgebung, wobei mikromechanische Systeme aufgrund ihrer geringen Wärmekapazität und ihrer vorzugsweise hohen Wärmeleitfähigkeit vorteilhaft sind.

Eine nutzergesteuerte Anpassung des Luftstroms 5, insbesondere des Massenstroms, der Aroma- und/oder Wirkstoffe und/oder der Temperatur kann durch eine schnelle Reaktionszeit des Verdampfers 3 ermöglicht werden. Die Verdampfung ist vorteilhaft mit einer Anpassung der Verdampfertemperatur, der Anzahl der aktivierten beziehungsweise angesteuerten Bereiche der Verdampfereinheit 20 beziehungsweise des oder der Verdampfer 3, 3a, 3b, 3c, 3d, einer Anpassung des Tastverhältnisses bei gepulster Ansteuerung und/oder einer Kombination davon einstellbar. Vorteilhaft können durch eine geeignete Kombination der Leistung P der Verdampfer 3, 3a, 3b, 3c, 3d, gegebenenfalls der Leistung P der Nacherwärmungsvorrichtung 10, des Massenstroms, der Temperaturdifferenzen ΔT vor und nach dem Verdampfer 3, 3a, 3b, 3c, 3d und/oder ggf. der Temperaturdifferenzen ΔT vor und nach der Nacherwärmungsvorrichtung 10 die Dampfmenge und der Aroma- und/oder Wirkstoffgehalt, insbesondere der Nikotingehalt, unabhängig voneinander variiert werden beziehungsweise variierbar sein.

An dem Gehäuse 28 ist vorteilhaft eine Anzeigevorrichtung 102 vorgesehen, die beispielsweise eine oder mehrere LEDs, Displays aus organischen Leuchtdioden (OLED) und/oder Flüssigkristalldisplays (LC-Displays) umfassen kann. Vorteilhaft kann die Anzeigevorrichtung 102 die Einstellung, insbesondere die Dampfmenge, Temperatur und/oder den Aroma- und/oder Wirkstoffgehalt, darstellen.

In Figur 5 ist eine Verdampfereinheit 20 gezeigt. Die Verdampfereinheit 20 umfasst einen blockförmigen, vorzugsweise monolithischen Heizkörper beziehungsweise Verdampfer 3 aus einem elektrisch leitenden Material, vorzugsweise dotiertes Silizium, dotierte Keramik, Metall-Keramik, Filter-Keramik, Halbleiter, insbesondere Germanium, Graphit, Halbmetall und/oder Metall. Es ist nicht erforderlich, dass der gesamte Verdampfer 3 aus einem elektrisch leitenden Material besteht. Es kann beispielsweise ausreichen, dass die Oberfläche des Verdampfers 3 elektrisch leitend, beispielsweise metallisch, beschichtet ist. In diesem Fall muss nicht die gesamte Oberfläche beschichtet sein, beispielsweise können Leiterbahnen auf einem nichtleitenden Grundkörper vorgesehen sein.

Der Verdampfer 3 ist mit einer Mehrzahl von Mikrokanälen 62 versehen, die eine Einlassseite 61 des Verdampfers 3 mit einer Auslassseite 64 flüssigkeitsleitend verbinden. Die Einlassseite 61 ist über eine Dochtstruktur 19 flüssigkeitsleitend mit dem Flüssigkeitsspeicher 18 verbunden. Die Dochtstruktur 19 dient zur passiven Förderung von Flüssigkeit aus einem Flüssigkeitsspeicher 18 zu dem Verdampfer 3 mittels Kapillarkräften. Im Kontaktbereich 61 zu dem Verdampfer 3 dient die Dochtstruktur 19 dazu, Flüssigkeit gleichmäßig zu verteilen, temperaturbeständig zu sein und mit ihren relativ kleinen Poren und/oder dünnen Kapillaren eine Art Rückschlagventil zu bilden, um unerwünschtes Rückfließen von blasenhaltiger Flüssigkeit aus dem Verdampfer 3 in die Dochtstruktur 19 und/oder in den Flüssigkeitsspeicher 18 zu verhindern.

Der mittlere Durchmesser der Mikrokanäle 62 liegt vorzugsweise im Bereich zwischen 5 µm und 200 µm, weiter vorzugsweise im Bereich zwischen 30 µm und 150 µm, noch weiter vorzugsweise im Bereich zwischen 50 µm und 100 µm. Aufgrund dieser Abmessungen wird vorteilhaft eine Kapillarwirkung erzeugt, so dass an der Einlassseite 61 in einen Mikrokanal 62 eindringende Flüssigkeit durch den Mikrokanal 62 nach oben steigt, bis der Mikrokanal 62 mit Flüssigkeit gefüllt ist. Das Volumenverhältnis von Mikrokanälen 62 zu Verdampfer 3, das als Porosität des Verdampfers 3 bezeichnet werden kann, liegt beispielsweise im Bereich zwischen 10% und 50%, vorteilhaft im Bereich zwischen 15% und 40%, noch weiter vorteilhaft im Bereich zwischen 20% und 30%, und beträgt beispielsweise 25%.

Die Kantenlängen der mit Mikrokanälen 62 versehenen Flächen des Verdampfers 3 liegen beispielsweise im Bereich zwischen 0,5 mm und 3 mm, vorzugsweise zwischen 0.5 mm und 1 mm. Die Abmessungen der mit Mikrokanälen 62 versehenen Flächen des Verdampfers 3 können beispielsweise betragen: 0,95 mm x 1,75 mm oder 1,9 mm x 1,75 mm oder 1,9 mm x 0,75 mm. Die Kantenlängen des Verdampfers 3 können beispielsweise im Bereich zwischen 0,5 mm und 5 mm, vorzugsweise im Bereich zwischen 0,75 mm und 4 mm, weiter vorzugsweise im Bereich zwischen 1 mm und 3 mm liegen. Die Fläche des Verdampfers 3 (chip size) kann beispielsweise 1 mm x 3 mm, 2mm x 2 mm oder 2 mm x 3 mm betragen.

Die Breite b des Verdampfers 3 (siehe Figur 5) liegt vorzugsweise im Bereich zwischen 1 mm und 5 mm, weiter vorzugsweise im Bereich zwischen 2 mm und 4 mm, und beträgt beispielsweise 3 mm. Die Höhe h des Verdampfers 3 (siehe Figur 5) liegt vorzugsweise im Bereich zwischen 0,05 mm und 1 mm, weiter vorzugsweise im Bereich zwischen 0,1 mm und 0,75 mm, noch weiter vorzugsweise im Bereich zwischen 0,2 mm und 0,5 mm und beträgt beispielsweise 0,3 mm.

Die Anzahl der Mikrokanäle 62 liegt vorzugsweise im Bereich zwischen vier und 1000. Auf diese Weise lässt sich der Wärmeeintrag in die Mikrokanäle 62 optimieren und eine gesicherte hohe Verdampfungsleistung sowie eine ausreichend große Dampfaustrittsfläche realisieren.

Die Mikrokanäle 62 sind in Form eines quadratischen, rechteckigen, vieleckigen, runden, ovalen oder anders geformten Arrays angeordnet. Das Array kann in Form einer Matrix mit s Spalten und z Zeilen ausgebildet sein, wobei s vorteilhaft im Bereich zwischen 2 und 50 und weiter vorteilhaft im Bereich zwischen 3 und 30 und/oder z vorteilhaft im Bereich zwischen 2 und 50 und weiter vorteilhaft im Bereich zwischen 3 und 30 liegt. Auf diese Weise lässt sich eine effektive und auf einfache Weise herstellbare Anordnung der Mikrokanäle 62 mit gesichert hoher Verdampfungsleistung realisieren.

Der Querschnitt der Mikrokanäle 62 kann quadratisch, rechteckig, vieleckig, rund, oval oder anders geformt sein, und/oder sich in Längsrichtung abschnittweise ändern, insbesondere vergrößern, verkleinern oder konstant bleiben.

Die Länge eines oder jedes Mikrokanals 62 liegt vorzugsweise im Bereich zwischen 100 µm und 1000 µm, weiter vorzugsweise im Bereich zwischen 150 µm und 750 µm, noch weiter vorzugsweise im Bereich zwischen 180 µm und 500 µm und beträgt beispielsweise 300 µm. Auf diese Weise lässt sich eine optimale Flüssigkeitsaufnahme und Portionsbildung bei ausreichend gutem Wärmeeintrag von dem Verdampfer 3 in die Mikrokanäle 62 realisieren.

Der Abstand zweier Mikrokanäle 62 beträgt vorzugsweise mindestens das 1,3-fache des lichten Durchmessers eines Mikrokanals 62, wobei der Abstand auf die Mittelachsen der beiden Mikrokanäle 62 bezogen ist. Der Abstand kann bevorzugt das 1,5- bis 5-fache, weiter bevorzugt das 2- bis 4-fache des lichten Durchmessers eines Mikrokanals 62 betragen. Auf diese Weise lässt sich ein optimaler Wärmeeintrag in die Mikrokanäle und eine ausreichend stabile Anordnung und Wandstärke der Mikrokanäle realisieren.

Die Verdampfereinheit 20 weist eine vorzugsweise von der Steuerungsvorrichtung 29 steuerbare Heizspannungsquelle 71 auf, die über Elektroden 72 an gegenüberliegenden Seiten des Verdampfers 3 mit diesem verbunden ist, so dass eine von der Heizspannungsquelle 71 erzeugte elektrische Spannung Uh zu einem Stromfluss durch den Verdampfer 3 führt. Aufgrund des Ohm'schen Widerstands des elektrisch leitenden Verdampfers 3 führt der Stromfluss zu einer Erhitzung des Verdampfers 3 und daher zu einer Verdampfung von in den Mikrokanälen 62 enthaltener Flüssigkeit. Auf diese Weise erzeugter Dampf/Aerosol 6 entweicht zur Auslassseite 64 aus den Mikrokanälen 62 und wird dem Luftstrom 5 beigemischt, siehe Figur 4. Genauer steuert bei Feststellung eines durch Ziehen des Konsumenten verursachten Luftstroms 5 durch den Luftkanal 30 die Steuerungsvorrichtung 29 die Heizspannungsquelle 71 an, wobei durch spontane Erhitzung die in den Mikrokanälen 62 befindliche Flüssigkeit in Form von Dampf/Aerosol 6 aus den Mikrokanälen 62 getrieben wird.

Dabei kann die Dauer der einzelnen Verdampfungsschritte bei unterschiedlichen Temperaturen und/oder einem Verdampfen der einzelnen Komponenten der einzelnen Portionen der Flüssigkeit derart kurz gehalten werden und/oder mit einer Ansteuerfrequenz getaktet erfolgen, dass die schrittweise Verdampfung von einem Konsumenten nicht wahrgenommen und trotzdem eine weitgehend homogene, geschmackskonforme, wiederholbar präzise Aerosolbildung gewährleistet werden kann. Insbesondere erfolgt vorteilhaft zunächst ein Verdampfen einer leichter siedenden Komponente der Flüssigkeit in einem ersten Verdampfungsintervall mit einer ersten Temperatur A und anschließend ein Verdampfen einer höher siedenden Komponente der Flüssigkeit in einem zweiten Verdampfungsintervall mit einer zweiten Temperatur B, welche die Temperatur A übersteigt. Bei der durch den Konsumenten vorgesehenen Anpassung der Aroma- und/oder Wirkstoffe hat die Temperatur einen Einfluss, da die unterschiedlichen Liquidbestandteile einen unterschiedlichen Dampfdruck aufweisen und deren relatives Verhältnis in der Dampfphase von der Temperatur abhängig ist.

Vorzugsweise ist in dem Datenspeicher des Inhalators 27 eine dem verwendeten Flüssigkeitsgemisch angepasste Spannungskurve Uh(t) hinterlegt. Dies ermöglicht es, den Spannungsverlauf Uh(t) dem verwendeten Liquid angepasst vorzugeben, so dass sich die Heiztemperatur des Verdampfers 3, und damit auch die Temperatur der kapillaren Mikrokanäle 62, gemäß der bekannten Verdampfungskinetik des jeweiligen Liquids zeitlich über den Verdampfungsvorgang steuern lässt, wodurch optimale Verdampfungsergebnisse erzielbar sind. Die Verdampfungstemperatur liegt vorzugsweise im Bereich zwischen 100°C und 400°C, weiter bevorzugt zwischen 150 °C und 350 °C, noch weiter bevorzugt zwischen 190 °C und 290 °C.

An der Einlassseite 61 des Verdampfers 3 ist vorteilhaft eine poröse und/oder kapillare, flüssigkeitsleitende Dochtstruktur 19 angeordnet. Die Dochtstruktur 19 kontaktiert die Einlassseite 61 des Verdampfers 3 flächig und deckt sämtliche Mikrokanäle 62 einlassseitig ab, wie in den Figur 5 ersichtlich ist. An der dem Verdampfer 3 gegenüberliegenden Seite ist die Dochtstruktur flüssigkeitsleitend mit dem Flüssigkeitsspeicher 18 verbunden. Die in den Figuren 4 und 5 gezeigte direkte Anbindung des Flüssigkeitsspeichers 18 an die Dochtstruktur 19 ist nur beispielhaft zu verstehen. Insbesondere können eine Flüssigkeitsschnittstelle und/oder eine mehrere Flüssigkeitsleitungen zwischen Flüssigkeitsspeicher 18 und Dochtstruktur 19 vorgesehen sein. Der Flüssigkeitsspeicher 18 kann daher auch beabstandet von der Dochtstruktur 19 angeordnet sein. Der Flüssigkeitsspeicher 18 kann in seinen Abmessungen größer als die Dochtstruktur 19 sein. Die Dochtstruktur 19 kann beispielsweise in eine Öffnung eines Gehäuses des Flüssigkeitsspeichers 18 eingesetzt sein. Es kann auch eine Mehrzahl von Verdampfereinheiten 20 einem Flüssigkeitsspeicher 18 zugeordnet sein. Die Dochtstruktur 19 kann generell einteilig oder mehrteilig sein.

Die Dochtstruktur 19 besteht aus porösem und/oder kapillarem Material, das aufgrund von Kapillarkräften in der Lage ist, von dem Verdampfer 3 verdampfte Flüssigkeit in ausreichender Menge von dem Flüssigkeitsspeicher 18 zu dem Verdampfer 3 passiv nachzufördern, um ein Leerlaufen der Mikrokanäle 62 und sich daraus ergebende Probleme zu verhindern. Die Dochtstruktur 19 besteht vorteilhaft aus einem nichtleitenden Material, um eine unerwünschte Erwärmung von Flüssigkeit in der Dochtstruktur 19 durch Stromfluss zu vermeiden. Falls die Dochtstruktur 19 aus einem leitenden Material besteht, was nicht ausgeschlossen ist, ist zwischen der Dochtstruktur 19 und dem Verdampfer 3 vorteilhaft eine Isolierschicht aus einem elektrisch und/oder thermisch isolierenden Material, beispielsweise Glas, Keramik oder Kunststoff, mit sich durch die Isolierschicht erstreckenden, mit den Mikrokanälen 62 korrespondierenden Durchgangsöffnungen vorgesehen.

Die Dochtstruktur 19 besteht vorteilhaft aus einem oder mehreren der Materialien Baumwolle, Cellulose, Acetat, Glasfasergewebe, Glasfaserkeramik, Sinterkeramik, keramisches Papier, Alumosilikat-Papier, Metallschaum, Metallschwamm, einem anderen hitzebeständigen, porösen und/oder kapillaren Material mit geeigneter Förderrate, oder einem Verbund von zwei oder mehr der vorgenannter Materialien. In einer vorteilhaften praktischen Ausführungsform kann die Dochtstruktur 19 mindestens ein Keramikfaserpapier und/oder eine poröse Keramik umfassen. Das Volumen der Dochtstruktur 19 liegt vorzugsweise im Bereich zwischen 1 mm³ und 10 mm³, weiter vorzugsweise im Bereich zwischen 2 mm³ und 8 mm³, noch weiter vorzugsweise im Bereich zwischen 3 mm³ und 7 mm³ und beträgt beispielsweise 5 mm³.

Der Verdampfer 3 kann vorteilhaft aus Teilstücken eines Wafers mit Dünnfilmschichttechnologie hergestellt werden, welcher eine Schichtdicke von vorzugsweise kleiner oder gleich 1000 µm, weiter vorzugsweise kleiner oder gleich 750 µm, noch weiter vorzugsweise kleiner oder gleich 500 µm aufweist. Oberflächen des Verdampfers 3 können vorteilhaft hydrophil sein. Die Einlassseite 61 und/oder die Auslassseite 64 des Verdampfers 3 kann vorteilhaft mikrostrukturiert sein bzw. Mikroausnehmungen (micro grooves) aufweisen.

Die Verdampfereinheit 20 ist so eingestellt, dass eine Flüssigkeitsmenge vorzugsweise im Bereich zwischen 1 µl und 20 µl, weiter vorzugsweise zwischen 2 µl und 10 µl, noch weiter vorzugsweise zwischen 3 µl und 5 µl, typischerweise 4 µl pro Zug des Konsumenten, zudosiert wird. Vorzugsweise kann die Verdampfereinheit 20 hinsichtlich der Flüssigkeits-/Dampfmenge pro Zug einstellbar sein.

Der Inhalator 27 umfasst vorteilhaft eine Durchfluss-Messanordnung 100 zur Messung des durch den Inhalator strömenden Luftvolumenstroms.

Eine vorteilhafte Durchfluss-Messanordnung 100 wird nachfolgend unter Bezugnahme auf Figur 6 beschrieben. Die Messanordnung beziehungsweise Durchfluss-Messanordnung 100 gemäß Figur 6 misst die Durchflussmenge des Luftstroms 5 beziehungsweise eines Messluftstroms 105 durch den Luftkanal 30 beziehungsweise einen Messluftkanal 104 auf thermischer Grundlage beziehungsweise nach dem Aufheizprinzip, wobei der Luftstrom 5 mittels einer Heizeinrichtung 63 um eine Temperaturdifferenz ΔT aufgeheizt wird.

Die Heizeinrichtung 63 kann beispielsweise von einem Verdampfer 3 oder einer Nacherwärmungsvorrichtung 10 gebildet sein. Dabei wird das thermische Verhalten des Verdampfers 3 und/oder der Nacherwärmungsvorrichtung 10, insbesondere eines Silizium-Heizers, genutzt. Das Messverfahren nach dem Aufheizprinzip kann demnach mit der Nacherwärmung des Luftstroms 5 durch die Nacherwärmungsvorrichtung 10 kombiniert werden. Vorteilhaft ist die Heizeinrichtung 63 jedoch zusätzlich zu, und unabhängig von, den Verdampfer 3 und den Nacherwärmungsvorrichtungen 10 vorgesehen, was eine genauere Durchflussmessung ermöglicht.

Der durch den Luftkanal 3 strömende Luftstrom 5 passiert eine Mehrzahl von Temperatursensoren 52, 53 zur Messung der Temperatur der den jeweiligen Temperatursensor 52, 53 passierenden Luft. Die Temperatursensoren 52, 53 sind in Strömungsrichtung voneinander beabstandet, d.h. sequenziell angeordnet. Ein vorgeschalteter Temperatursensor 52 ist der Heizeinrichtung 63 vorgeschaltet, d.h. stromaufwärts von der Heizeinrichtung 63 angeordnet. Ein nachgeschalteter Temperatursensor 53 ist der Heizeinrichtung 63 nachgeschaltet, d.h. stromabwärts von der Heizeinrichtung 63 angeordnet. Die Temperatursensoren 52, 53 geben Messtemperatursignale an eine Auswerteschaltung 51 und/oder die elektronische Steuerungsvorrichtung 29 des Inhalators 27 aus.

Im Beispiel der Figur 6 ist eine Auswerteschaltung 51 zur Bestimmung der Temperaturdifferenz ΔT zwischen den mit den Temperatursensoren 52, 53 gemessenen Temperaturen vorgesehen. Diese Auswertung kann selbstverständlich auch in der elektronischen Steuerungsvorrichtung 29 erfolgen, so dass eine separate Auswerteschaltung 51 entbehrlich sein kann.

Die Heizeinrichtung 63 erwärmt den Luftstrom 5 im Luftkanal 30 mit einer elektrischen Heizleistung P. Die Messanordnung beziehungsweise Durchfluss-Messanordnung 100 umfasst vorzugsweise ein Wattmeter 103 zur Messung der von der Heizeinrichtung 103 erzeugten und an den Luftstrom 5 abgegebenen Leistung P. Das Wattmeter 103 kann eine separate Schaltung sein. Alternativ kann die Heizleistung aus einer Steuerelektronik für die Heizeinrichtung 63 bekannt sein und an die elektronische Steuerungsvorrichtung 29 übermittelt werden. Das Wattmeter 103 kann somit in einer Steuerelektronik für die Heizeinrichtung 63 und/oder in der elektronischen Steuerungsvorrichtung 29 realisiert sein.

Die dem Luftstrom 5 zugeführte Wärme erhöht dessen Temperatur. Der vorgeschaltete Temperatursensor 52 dient zur Messung der Temperatur des Luftstroms 5 stromaufwärts des Verdampfers 3 beziehungsweise im Luftstrom 5 vor der Erwärmung durch die Heizeinrichtung 63. Der nachgeschaltete Temperatursensor 53 dient zur Messung der Temperatur des Luftstroms 5 stromabwärts der Heizeinrichtung 63 beziehungsweise im Luftstrom 5 nach der Erwärmung durch die Heizeinrichtung 63.

Die elektronische Steuerungsvorrichtung 29 ermittelt den Massenstrom Δm / Δt des durch den Luftkanal 30 strömenden Luftstroms 5 mittels der Gleichung Δm / Δt = P/(c·ΔT), wobei P die mit dem Wattmeter 103 gemessene Heizleistung, ΔT die Temperaturdifferenz insbesondere zwischen den Temperatursensoren 52, 53 und c die bekannte Wärmekapazität der Luft bzw. des Luft-/Aerosolgemisches ist. Der Volumenstrom des Luftstroms 5 durch den Luftkanal 30 hängt durch eine bekannte Funktion oder eine Proportionalitätskonstante, die beispielweise per Kalibrierung ermittelbar und in der elektronischen Steuerungsvorrichtung 29 speicherbar ist, mit dem Massenstrom zusammen und ist bei Bedarf einfach von der elektronischen Steuerungsvorrichtung 29 ermittelbar.

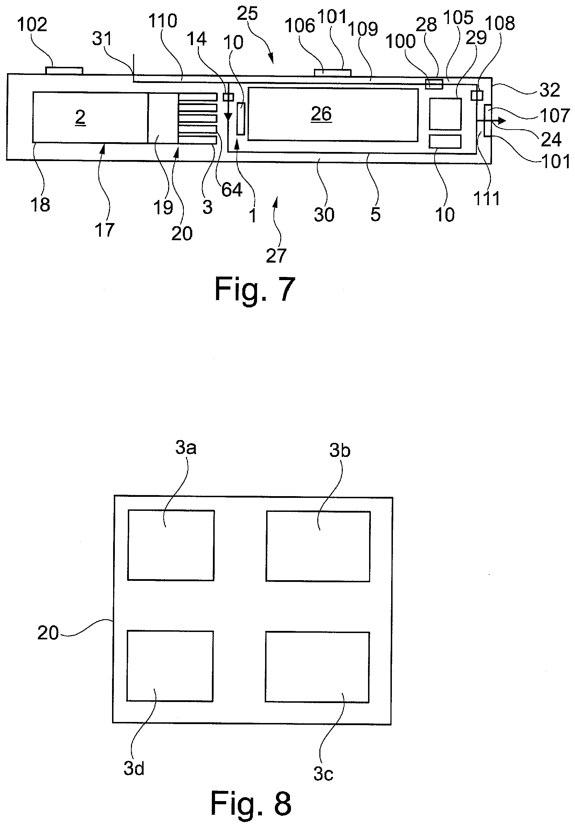

Vorzugsweise sind beide Temperatursensoren 52, 53 und die Heizeinrichtung 63 der Durchfluss-Messanordnung 100 vor dem ersten Verdampfer 3 oder in einem als By-Pass 109 vorgesehenen Messkanal 104 angeordnet, wie beispielsweise in Figur 4 beziehungsweise in Figur 7 gezeigt. In diesen Fällen kann als Wärmekapazität c die konstante Wärmekapazität von Luft ohne Dampf bzw. Aerosol verwendet werden, was die Genauigkeit der Messung erhöht. Es ist aber auch denkbar, dass die Durchfluss-Messanordnung 100 ganz oder teilweise hinter dem ersten Verdampfer 3 angeordnet ist. Dies ist insbesondere dann der Fall, wenn die Heizeinrichtung 63 von einem Verdampfer 3 und/oder einer Nacherwärmungseinrichtung 10 gebildet ist. In diesem Fall ist als Wärmekapazität c die Wärmekapazität des Luft-/Dampf-/Aerosolgemisches anzunehmen.

Vorzugsweise ist die Durchfluss-Messanordnung 100 zur zeitaufgelösten Messung der Zugstärke durch den Nutzer eingerichtet. Besonders bevorzugt ist die Verdampferleistung des Verdampfers 3, dessen Dampfrate variierbar ist, anhand der zeitaufgelösten Messung an die Zugstärke des Konsumenten anpassbar. Insbesondere ist vorteilhaft der Tastgrad und/oder die Temperatur des Verdampfers 3 einstellbar, um den Aroma- und/oder Wirkstoffanteil, insbesondere den Nikotinanteil, im Aerosol 6 beziehungsweise in der Dampfphase anhand der zeitaufgelösten Messung der Messanordnung 100 an die Zugstärke anzupassen.

Die Durchfluss-Messanordnung 100 kann vorteilhaft zur Aktivierung des Inhalators 27 genutzt werden, indem der Inhalator 27 beispielsweise bei einem Pusten des Konsumenten aktiviert wird, um eine fehlerfreie Erkennung der Aktivierung durch den Konsumenten feststellen zu können. Die Steuerungsvorrichtung 29 kann das Pusten des Konsumenten in ein Steuerungssignal für beispielsweise die Verdampfereinheit 20 umwandeln, um beispielsweise durch ein bestimmtes Pusten eine vorbestimmte Dampfmenge einzustellen.

Bevorzugt kann der Inhalator 27 ausschließlich durch von der Durchfluss-Messanordnung 100 beziehungsweise Druck- oder Strömungssensoren gemessenes Blasen und/oder Ziehen durch den Nutzer einstellbar, steuerbar beziehungsweise regelbar sein, sodass Schalter, Taster oder ähnliches entbehrlich sind. Beispielhaft kann ein langes Blasen das Aufrufen eines Einstellungsmenüs bedeuten, wobei durch kurzes Blasen und/oder kurzes Ziehen eine oder mehrere Einstellungen veränderbar sind. Die Einstellung kann beispielsweise durch ein weiteres langes Blasen und/oder nach einer definierten Zeit abgeschlossen werden. Die Verdampfungsmenge ist demnach vorteilhaft durch den Zugwiderstand beziehungsweise durch den von dem Konsumenten beaufschlagten Saugdruck steuerbar beziehungsweise regelbar.

Figur 7 zeigt einen Inhalator 27, welcher in Hinblick auf die Unterschiede zu der in Figur 4 gezeigten Ausführungsform erläutert wird. Der in Figur 7 gezeigte Inhalator 27 umfasst einen Bypass-Kanal oder Nebenluftkanal 109. Die Durchfluss-Messanordnung 100 (d.h. der nachgeschaltete Temperatursensor 53, ggf. der vorgeschaltete Temperatursensor 52 und/oder die Heizeinrichtung 63) ist in dem Bypass-Kanal oder Nebenluftkanal 109 vorteilhaft ohne Verdampfer 3 angeordnet. Dies hat ebenso wie die in Figur 4 gezeigte Anordnung den Vorteil, dass die Durchflussmessung nicht von dem Dampf-/Aerosolgehalt in dem Luftstrom 5 beziehungsweise Messluftstrom 105 beeinflusst wird.

Der Bypasskanal 109 ist ein von dem Luftkanal 30 wenigstens abschnittsweise verschiedener Kanal. Vorteilhaft ist stromaufwärts vom Verdampfer 3 beziehungsweise zwischen der Einlassöffnung 31 und dem Verdampfer 3 eine Abzweigung 110 vorgesehen, an dem sich der Bypasskanal 109 und der Luftkanal 30 trennen. Der Bypasskanal 109 und der Luftkanal 30 erstrecken sich parallel zueinander bis zu einem Knoten 111, an dem sie vorteilhaft zusammen einen gemeinsamen Luftstrom bilden, der zu der Luftauslassöffnung 24 geführt wird und insbesondere von dem Konsumenten inhaliert werden kann. Der Bypasskanal 109 dient vorteilhaft der Umgehung der Verdampfereinheit 20 beziehungsweise des Verdampfers 3.

Der Bypasskanal 109 kann vorteilhaft eine Blende 108 oder ein Ventil umfassen, wobei die Blende 108 dazu eingerichtet ist, den Bypasskanal 109 zu schließen bzw. zu öffnen, d.h. den Bypasskanal 109 für einen Luftstrom passierbar oder unpassierbar zu machen. Damit kann beispielsweise die Strömungsverhältnisse an dem mindestens einen Verdampfer geregelt und/oder der Strömungswiderstand im Inhalator 27 beeinflusst werden.

In einer nicht gezeigten Ausführungsform ist in dem Bypasskanal 109 eine wie in der Beschreibung zu Figur 1 erläuterte Drossel 14 beispielsweise zur Regelung/Steuerung des Strömungswiderstandes in dem Bypasskanal angeordnet.

In der in Figur 7 gezeigten Ausführungsform bildet der Bypasskanal 109 den in Figur 6 gezeigten Messkanal 104, der den Messluftstrom 105 zur Durchflussmessung mit der Durchfluss-Messanordnung 100 führt.

Figur 8 zeigt eine mehrkanalige Verdampfereinheit 20 mit einer Mehrzahl, hier vier, Verdampfern 3a, 3b, 3c, 3d. Vorteilhaft sind die Verdampfer 3a, 3b, 3c, 3d separat voneinander beheizbar beziehungsweise ansteuerbar, was eine schnelle Reaktionszeit der Verdampfungsleistung der Verdampfereinheit 20 ermöglicht. Die Verdampfer 3a, 3b, 3c, 3d können mit einem oder mehreren Flüssigkeitsspeicher 18 verbunden und/oder verbindbar sein. Damit können beispielsweise zwei der Verdampfer 3a, 3b, 3c, 3d unterschiedliche Flüssigkeiten 2 mit unterschiedlichen Aroma- und/oder Wirkstoffen verdampfen und dem Luftstrom 5 zugeben.

Einzelne Verdampfer 3a, 3b, 3c, 3d (Figur 8), alternativ einzelne Verdampferchips und/oder Bereiche eines Verdampfers 3 können vorteilhaft separat voneinander angesteuert, insbesondere an- und abgeschaltet, d.h. aktiviert werden. Somit kann die Dampfmenge beziehungsweise der Massenstrom durch die Ansteuerimpulse und/oder mit der Konstellation der angesteuerten Verdampfer 3a, 3b, 3c, 3d oder Bereiche eines Verdampfers 3 eingestellt beziehungsweise geregelt werden. Beispielsweise kann eine Abstufung bei der Regelung der Ansteuerung der Verdampfer 3 oder Bereiche eines Verdampfers 3 vorgesehen sein, bei der verschiedene Modi denkbar sind. Beispielsweise können für eine schwache beziehungsweise wenig Verdampfung zwei Verdampfer 3a, 3b, für eine Standardmenge beziehungsweise durchschnittliche Menge drei Verdampfer 3a, 3b, 3c und für eine starke beziehungsweise viel Verdampfung vier Verdampfer 3a, 3b, 3c, 3d, oder alternativ Bereiche eines Verdampfers 3, aktivierbar sein.

## Patentansprüche

1. Anordnung (1) für einen Inhalator (27), umfassend
- mindestens einen elektrischen Verdampfer (3, 3a, 3b, 3c, 3d) zum Verdampfen von dem Verdampfer (3, 3a, 3b, 3c, 3d) zugeführter Flüssigkeit (2) und zur Zugabe der verdampften Flüssigkeit (2) zu einem durch den Inhalator (27) strömenden Luftstrom (5) zur Bildung eines Aerosols (6), und
- eine Durchfluss-Messanordnung (100) zur Messung des Volumen- und/oder Massenstroms des durch den Inhalator (27) strömenden Luftstroms (5),
**dadurch gekennzeichnet, dass**
- die Durchfluss-Messanordnung (100) eine Heizeinrichtung (63), einen stromabwärts der Heizeinrichtung (63) angeordneten nachgeschalteten Temperatursensor (53) zur Messung einer Luftausgangstemperatur und eine elektronische Steuerungsvorrichtung (29) umfasst, und
- die elektronische Steuerungsvorrichtung (29) zur Ermittlung des Volumen- und/oder Massenstroms des durch den Inhalator (27) strömenden Luftstroms (5) auf der Grundlage einer Temperaturdifferenz zwischen der Luftausgangstemperatur und einer Lufteingangstemperatur des Luftstroms (5) stromaufwärts der Heizeinrichtung (63) eingerichtet ist.

2. Anordnung (1) nach Anspruch 1, **dadurch gekennzeichnet, dass**
- die Durchfluss-Messanordnung (100) einen stromaufwärts der Heizeinrichtung (63) angeordneten vorgeschalteter Temperatursensor (52) zur Messung der Lufteingangstemperatur des Luftstroms (5) umfasst.

3. Anordnung (1) nach einem der vorangehenden Ansprüche, **dadurch gekennzeichnet, dass**
- die Heizeinrichtung (63) stromaufwärts von dem oder den Verdampfern (3, 3a, 3b, 3c, 3d) des Inhalators (27) angeordnet ist.

4. Anordnung (1) nach einem der vorangehenden Ansprüche, **dadurch gekennzeichnet, dass**
- die Heizeinrichtung (63) von einem der Verdampfer (3, 3a, 3b, 3c, 3d) gebildet ist.

5. Anordnung (1) vorzugsweise nach einem der vorangehenden Ansprüche, aufweisend mindestens einen elektrischen Verdampfer (3, 3a, 3b, 3c, 3d) zum Verdampfen von dem Verdampfer (3, 3a, 3b, 3c, 3d) zugeführter Flüssigkeit (2) und zur Zugabe der verdampften Flüssigkeit (2) zu einem durch den Inhalator (27) strömenden Luftstrom (5) zur Bildung eines Aerosols (6), **dadurch gekennzeichnet, dass**
- die Anordnung (1) eine Nacherwärmungsvorrichtung (10) zur Nacherwärmung des Aerosols (6) umfasst.

6. Anordnung nach Anspruch 5, **dadurch gekennzeichnet, dass**
- die Heizeinrichtung (63) von der Nacherwärmungsvorrichtung (10) gebildet ist.

7. Anordnung (1) nach einem der vorangehenden Ansprüche, **dadurch gekennzeichnet, dass**
- die elektronische Steuerungsvorrichtung (29) zur Ermittlung des Volumen- und/oder Massenstroms des durch den Inhalator (27) strömenden Luftstroms (5) durch Anwendung der Gleichung Δm / Δt = P/(c·ΔT) eingerichtet ist, wobei Δ m / Δ t der Massenstrom, P die Heizleistung der Heizeinrichtung (63), ΔT die Temperaturdifferenz zwischen der Luftausgangstemperatur und der Lufteingangstemperatur und c die Wärmekapazität der durch die Heizeinrichtung (63) erwärmten Luft sind.

8. Anordnung (1) nach einem der vorangehenden Ansprüche, **dadurch gekennzeichnet, dass**
- die Anordnung (1) ein Wattmeter (103) zur Ermittlung der Heizleistung P der Heizeinrichtung (63) umfasst.

9. Anordnung (1) nach einem der vorangehenden Ansprüche, **dadurch gekennzeichnet, dass**
- die elektronische Steuerungsvorrichtung (29) zur Steuerung des mindestens einen Verdampfers (3, 3a, 3b, 3c, 3d) in Abhängigkeit der gemessenen Durchflussmenge des Luftstroms (5) durch den Inhalator (27) eingerichtet ist.

10. Anordnung (1) nach einem der vorangehenden Ansprüche, **dadurch gekennzeichnet, dass**
- der Verdampfer (3, 3a, 3b, 3c, 3d) auf der Grundlage eines Signals von einem Betätigungselement (101) steuerbar ist.

11. Anordnung (1) nach einem der vorangehenden Ansprüche, **dadurch gekennzeichnet, dass**
- mindestens die Heizeinrichtung (63) und der nachgeschaltete Temperatursensor (53) in einem Messkanal (104) angeordnet ist.

12. Anordnung (1) nach einem der vorangehenden Ansprüche, **dadurch gekennzeichnet, dass**
- der mindestens eine elektrische Verdampfer (3, 3a, 3b, 3c, 3d) ein mehrkanaliger, insbesondere temperaturgesteuerter Verdampfer (3, 3a, 3b, 3c, 3d) ist.

13. Anordnung (1) nach einem der vorangehenden Ansprüche, **dadurch gekennzeichnet, dass**
- die Verdampfungsmenge der verdampften Flüssigkeit (2) einstellbar, steuerbar und/oder regelbar ist.

14. Anordnung (1) nach einem der vorangehenden Ansprüche, **dadurch gekennzeichnet, dass**
- die Nikotinmenge der verdampften Flüssigkeit (2) einstellbar, steuerbar und/oder regelbar ist.

15. Anordnung (1) nach einem der vorangehenden Ansprüche, **dadurch gekennzeichnet, dass**
- die Temperatur des Verdampfers (3, 3a, 3b, 3c, 3d) einstellbar, steuerbar und/oder regelbar ist.

16. Anordnung (1) nach einem der vorangehenden Ansprüche, **dadurch gekennzeichnet, dass**
- die Anzahl der aktivierten Verdampfer (3, 3a, 3b, 3c, 3d) und/oder Verdampferbereiche einstellbar, steuerbar und/oder regelbar ist.

17. Anordnung (1) nach einem der vorangehenden Ansprüche, **dadurch gekennzeichnet, dass**
- der Tastgrad des Verdampfers (3, 3a, 3b, 3c, 3d) einstellbar, steuerbar und/oder regelbar ist.

18. Anordnung (1) nach einem der vorangehenden Ansprüche, **dadurch gekennzeichnet, dass**
- der Verdampfer (3, 3a, 3b, 3c, 3d) wenigstens teilweise siliziumbasiert, vorzugsweise ein Mikro-Elektro-Mechanisches System (MEMS), ist.

19. Basisteil (25) für einen Inhalator (27), umfassend eine Anordnung (1) nach einem der vorangehenden Ansprüche.

20. Inhalator (27) mit einer Anordnung (1) nach einem der Ansprüche 1 bis 19.
